# EUROPEAN PATENT APPLICATION

(11) **EP 2 738 198 A1**
(43) Date of publication of application: **04.06.2014**
(21) Application number: 12008024.7
(22) Date of filing: 29.11.2012
(51) Int. Cl.: C08G 69/28, C08G 69/40, C12P 13/02, C12N 9/18, C12N 9/20

(54) **Enzymatic synthesis of polyamide in aqueous mini-emulsion**

(71) Applicant: Henkel AG & Co. KGaA, 40589 Düsseldorf (DE); Max-Planck-Gesellschaft zur Förderung der Wissenschaften e.V., 80539 München (DE)
(72) Inventor: Dr. Andreas Taden, 40597 Düsseldorf (DE); Dr Ito Kenji, 659-0041 Hyogo (JP); Prof. Dr. Katharina Landfester, 55122 Mainz (DE)

(57) **Abstract**

A process for preparing an aqueous polyamide dispersion, said process comprising reacting in an aqueous medium: a) an organic diamine compound (A) and b) an organic dicarboxylic acid compound (B), in the presence of c) at least one hydrolase enzyme (C) which catalyzes a polycondensation reaction of the diamine compound (A) and dicarboxylic acid compound (B) and d) an emulsifier (D), said process being characterized in that at least a fraction of the diamine compound (A) and the dicarboxylic acid compound (B) are provided in the aqueous medium as a disperse phase having an average droplet diameter of from 40 to 500nm.

## Description

### Field of the Invention

This invention relates to polyamides and processes for making and using those polyamides. More particularly, the polyamide is formed by an aqueous mini-emulsion polymerization process in which the polycondensation reaction between a polyamine and a polycarboxylic acid is enzyme catalyzed.

### Background of the Invention

Polyamides are extensively used as components in, for example, adhesives, sealants, coating compositions, paints and cosmetic formulations. At the present time it is more usual for polyamides to be synthesized via chemical processes using chemical catalysts at high temperatures and in the presence of organic solvents. Broadly, the main polyamine production processes employed in industry are: amidation of dicarboxylic acid compounds with diamines; self-amidation of amino acids; and, ring opening polymerization of lactams.

The synthesis of polyamides via chemically catalyzed polycondensation reactions between polyamine compounds and polycarboxylic acid compounds has well been documented in the art, such as in: US Patent No. 2,951,054 (Gen Dispersions Inc.); US Patent No. 3,130,181 (Allied Chemical); US Patent No. 4,886,844 (Union Camp Corporation); and, US Patent No. 6,777,488 (Sumitomo Seika Chemicals). That said, its has now been recognized that these typically multistage, complex chemical processes are energetically inefficient, particularly as the polymerizations are usually carried out at temperatures greater than 200°C. Moreover, these chemically catalyzed methods have often failed to provide relatively pure, high molecular weight polyamides at high yields. As a consequence, there has in recent times been some focus on enzymatic catalysis of polycondensation reactions.

WO94/12652 (Baxenden Chemicals Limited) describes a process for producing a polyesteramide comprising as repeating units residues of (a) at least one aliphatic dicarboxylic acid or derivative thereof, (b) at least one aliphatic hydroxyamine, diol, polyol, diamine or polyamine, and optionally (c) at least one aliphatic hydroxycarboxylic acid, aminocarboxylic acid or derivative thereof wherein said process the above defined components in the absence of a solvent and in the presence of a lipase.

WO01/96588 (Hercules Incorporated) describes a process for preparing a polyamide as an enzymatic reaction product of at least one polyamine and a diester. Suitable hydrolytic enzymes for the process may be free or immobilized and selected from lipase, esterase and protease.

US Patent Application Publication No. 2008/0275182 (Kong et al., assigned to BASF AG) describes a process for preparing an aqueous polyamide dispersion which comprises reacting, in an aqueous medium: a) an organic diamine compound A; and b) an organic dicarboxylic acid compound B, in the presence of c) of an enzyme C which catalyzes a polycondensation reaction of diamine compound A and dicarboxylic acid B, d) a dispersant D, and if appropriate, e) a low water solubility organic solvent E. In an embodiment, at least a portion of the diamine compound, the dicarboxylic acid compound and / or the solvent is present in the aqueous medium as a disperse phase having an average droplet size of less than or equal to 1000nm. In all the Examples of this document the reacted diamine is 3,3'-dimethly-4,4'-diaminocyclohexylmethane and the average droplet size of the disperse phase is not more specifically defined.

Ragupathy et al. in Journal of Molecular Catalysis B, Enzymatic, Volume 76, April 2012, pages 94-105 discloses a Novozyme-435 (N-435) catalyzed polycondensation reaction between different aliphatic (oligo)esters and diamines by precipitation polymerization at elevated temperatures which yields various particulate polyamides and poly(amide-co-ester). Two and three step synthetic methods to produce different polyamides such as nylon-8,10, nylon-8,13, nylon-6,13, and nylon-12,13 are provided. These very recent methods are not performed via emulsion polymerization.

There remains a need in the art to provide a method of producing polyamides at good yields via an enzymatic polymerization which can be performed under mild reaction conditions without using toxic reagents. Moreover, there is a need to demonstrate an enzymatic polycondensation process for the production of polyamides which can be performed reliably using a number of different diamines in the enzyme substrate.

### Statement of the Invention

In accordance with a first aspect of the invention there is provided a process for preparing an aqueous polyamide dispersion, said process comprising reacting in an aqueous medium:
a) an organic diamine compound (A) and
b) an organic dicarboxylic acid compound (B),
   in the presence of
c) of at least one hydrolase enzyme (C) which catalyzes a polycondensation reaction of the diamine compound (A) and dicarboxylic acid compound (B) and
d) an emulsifier (D)
said process being characterized in that at least a fraction of the diamine compound (A) and the dicarboxylic acid compound (B) are provided in the aqueous medium as a disperse phase having an average droplet diameter of from 40 to 500nm.

The molar ratio of the dicarboxylic acid compound (B) to the organic diamine compound (A) in the aqueous, reaction medium is generally from (0.5-1.5): 1, preferably from (0.8-1.2): 1, and more preferably from (0.95-1.05):1, for example 1:1.

This combination of process features is not explicitly taught in the enabled embodiments, or examples, of US Patent Application Publication No. 2008/0275182 (Kong et al., assigned to BASF AG). By selecting this combination of process features, the present invention can utilize organic diamine compounds not previously envisaged in enzymatic polycondensation reactions. In particular, the organic diamine compound (A) may be selected from the group consisting of fatty dimer amines and poly(alkylene oxide) diamines having terminal, primary amine groups.

The dicarboxylic acid compound (B) is preferably selected from the group consisting of: hexanedioic acid; heptanedioic acid, octanedioic acid; nonanedioic acid; decanedioic acid; undecanedioic acid; dodecanedioc acid; and, the corresponding C₄ to C₁₂ alkyl diesters thereof. More preferably, the dicarboxylic acid compound (B) has the general Formula:

(I) CₙH₂ₙ₊₁OOC(CH₂)ₘCOOCₙH₂ₙ₊₁,

wherein: m is an integer from 6 to 10; and, n is an integer from 5 to 11.

The total amount of hydrolase enzyme (C) in the aqueous medium is usually from 0.001 to 40% by weight, preferably from 0.1 to 15% by weight and more preferably from 0.5 to 8% by weight, based on the total amounts of diamine compound (A) and dicarboxylic acid compound (B) in said medium. In an embodiment, the hydrolase enzyme (C) is an esterase [EC 3.1.x.x] and preferably an esterase selected from carboxylesterases [EC 3.1.1.x] and lipases [EC 3.1.1.3].

The aqueous medium may further comprise up to 10 parts by weight, based on 100 parts by weight of water, of an organic solvent having a logarithm of the octanol-water partition coefficient (log P) greater than 2.

In accordance with a second aspect of the invention, there is provided an aqueous polyamide dispersion obtainable by the process according to any one of claims 1 to 9.

In accordance with a third aspect of the invention, there is provided a mini-emulsion comprising: an emulsifier (D); a disperse phase comprising an organic diamine compound (A) and a carboxylic acid compound (B); and, a continuous phase comprising water, wherein said disperse phase has an average droplet diameter of from 40 to 500nm, preferably from 100 to 300nm.

In an embodiment of this mini-emulsion which is intended for use in preparing an aqueous dispersion of a polyamide via an enzyme catalyzed polycondensation reaction: said organic diamine compound (A) is selected from the group of aliphatic diamines consisting of 1,6-diaminohexane, 1,7-diaminoheptane, 1,8-diaminooctane, 1,9-diaminononane, 1,10-diaminodecane, 1,11-diaminoundecane, 1,12-diaminododecane and, mixtures thereof; and said dicarboxylic acid compound (B) has the general Formula (I) CₙH₂ₙ₊₁OOC(CH₂)ₘCOOCₙH₂ₙ₊₁, in which m is an integer from 6 to 10 and n is an integer from 5 to 11.

In an alternative embodiment of this mini-emulsion intended for the same purpose: said organic diamine compound (A) is selected from the group consisting of fatty dimer amines and poly(alkylene oxide) diamines having terminal, primary amine groups; and said dicarboxylic acid compound (B) has the general Formula (I) CₙH₂ₙ₊₁OOC(CH₂)ₘCOOCₙH₂ₙ₊₁, in which m is an integer from 6 to 10 and n is an integer from 5 to 11.

### Description of the Drawings

The invention will be described with reference to the appended drawings in which:
Fig. 1 proposes a scheme for the lipase-catalyzed polycondensation of diester with diamine within an aqueous mini-emulsion.
Fig. 2 is a ¹H Nuclear Magnetic Resonance (NMR) spectrum of the crystalline product of a lipase-catalyzed polycondensation of diethyl sebacate with 1,8-diaminooctane as performed in accordance with an embodiment of the present invention.
Fig. 3 is an Infrared (IR) Spectrum of a crystalline product of lipase-catalyzed polycondensation of diethyl sebacate with 1,8-diaminooctane as performed in accordance with an embodiment of the present invention.

### Definitions

As used herein and in the appended claims, the terms "sonicate" or "sonication" will be understood to mean a process of exposing a suspension to the disruptive effect of the energy of high frequency sound waves.

The average size of the droplets of the dispersed phase of the aqueous miniemulsion and the average size of the derived polyamide particles are determined in accordance with the principle of quasielastic dynamic light scattering. The so-called z-average droplet diameter (d_{z}) of the unimodal analysis of the autocorrelation function is obtained using a Submicron Particle Sizer NICOMP TM available from PSS NICOMP Particle Sizing Systems. The measurements were performed at 1 atmosphere pressure and 25°C on dilute aqueous emulsions. As regards the mini-emulsion, between 0.1 and 1 g of the aqueous mini-emulsion was diluted with 1000 g of water, which had been saturated beforehand with the reacting monomers comprised in the aqueous miniemulsion, and to which thereafter 5% by weight of the dispersant used was added; the purpose of these measures is to prevent dilution being accompanied by a change in droplet diameter.

### Detailed Description of the Invention

The water which used to form the aqueous medium of the present invention may be provided by clear water of drinking water quality but should ideally be deionized water. The amount of water in the aqueous medium should be at least 80 wt.-%, preferably at least 90 wt.-%, and more preferably at least 95 wt.-%, and particularly preferably at least 99.5 wt.-%, , each based on the total amount of the aqueous medium. In some embodiments of the present invention the aqueous medium may additionally comprise water-miscible organic solvents, such as ethanol and/or acetone.

### Organic Diamine Compounds (A)

The organic diamine compounds (A) typically employed in the present invention should have two primary or secondary amino groups, of which preference is given to primary amino groups. The basic organic skeleton having the two amino groups may have an aliphatic, cycloaliphatic, aromatic or heteroaromatic structure. And mixtures of such diamine compounds are not precluded by the present invention.

Examples of compounds having two primary amino groups which may be used alone or in combination include 1,2-diaminoethane, 1,3-diaminopropane, 1,2-diaminopropane, 2-methyl-1,3-diaminopropane, 2,2-dimethyl-1,3-diaminopropane (neopentyldiamine), 1,4-diaminobutane, 1,2-diaminobutane, 1,3-diaminobutane, 1-methyl-1,4-diaminobutane, 2-methyl-1,4-diaminobutane, 2,2-dimethyl-1,4-diaminobutane, 2,3-dimethyl-1,4-diaminobutane, 1,5-diaminopentane, 1,2-diaminopentane, 1,3-diaminopentane, 1,4-diaminopentane, 2-methyl-1,5-diaminopentane, 3-methyl-1,5-diaminopentane, 2,2-dimethyl-1,5-diaminopentane, 2,3-dimethyl-1,5-diaminopentane, 2,4-dimethyl-1,5-diaminopentane, 1,6-diaminohexane, 1,2-diaminohexane, 1,3-diaminohexane, 1,4-diaminohexane, 1,5-diaminohexane, 2-methyl-1,5-diaminohexane, 3-methyl-1,5-diaminohexane, 2,2-dimethyl-1,5-diaminohexane, 2,3-dimethyl-1,5-diaminohexane, 3,3-dimethyl-1,5-diaminohexane, N,N'-dimethyl-1,6-diaminohexane, 1,7-diaminoheptane 1,8-diaminooctane, 1,9-diaminononane, 1,10-diaminodecane, 1,11-diaminoundecane, 1,12-diaminododecane, 1,2-diaminocyclohexane, 1,3-diaminocyclohexane, 1,4-diaminocyclohexane, 3,3'-diaminodicyclohexylmethane, 4,4'-diaminodicyclohexylmethane (dicyan), 3,3'-dimethyl-4,4'-diaminodicyclohexylmethane (Laromin(R)), isophoronediamine (3-aminomethyl-3,5,5-trimethyl-cyclohexylamine), 1,4-diazine (piperazine), 1,2-diaminobenzene, 1,3-diaminobenzene, 1,4-diaminobenzene, m-xylylenediamine [1,3-(diaminomethyl)benzene] and, p-xylylenediamine [1,4-(diaminomethyl)benzene].

In accordance with a first embodiment of the invention, the organic diamine compound (A) may be selected from the group of aliphatic diamines consisting of: 1,6-diaminohexane; 1,7-diaminoheptane; 1,8-diaminooctane; 1,9-diaminononane; 1,10-diaminodecane; 1,11-diaminoundecane; 1,12-diaminododecane; and, mixtures thereof.

Unexpectedly, the authors have also found that organic diamine compounds having molecular weights greater than 200, for example greater than 230 or even 400 and which possess two primary, preferably terminal amine groups can be used in the present invention.

A first group of such amines consists of fatty dimer amines of which may be mentioned the commercially available hydrophobic amines: VERSAMINE® 552 hydrogenated fatty C36 dimer diamine, VERSAMINE® 551 fatty C36 dimer diamine (both available from Cognis Corporation), PRIAMINE® 1073 and PRIAMINE®1074 fatty C36 dimer diamine (both available from Croda International Plc.). Advantageously these fatty dimer diamines can be prepared commercially from fatty dimer diacids which have been produced from dimerisation of vegetable oleic acid or tall oil fatty acid by thermal or acid catalyzed methods. In other words, these fatty dimer diamines can be derived from renewable sources.

A second group of such amines consists of poly(alkylene oxide) diamines which comprise hydrophilic polymer chains in addition to the terminal amine groups. The commercial diamines JEFFAMINE® D-230, JEFFAMINE® D-400, JEFFAMINE® D-2000, JEFFAMINE® D-4000, JEFFAMINE® T-403, JEFFAMINE® EDR-148, JEFFAMINE® EDR-192, JEFFAMINE® C-346, JEFFAMINE® ED-600, JEFFAMINE® ED-900, JEFFAMINE® ED-2001 may be specifically mentioned.

### Dicarboxylic Acid Compounds (B)

The organic dicarboxylic acid compounds (B) as used in the present invention may generally be any C₂-C₄₀-aliphatic, C₃-C₂₀-cycloaliphatic, aromatic or hetero-aromatic compounds which have two carboxylic acid groups (carboxyl groups), or derivatives thereof. Mixtures of such carboxylic acid compounds are not precluded.

Non-limiting examples of such compounds, which may be used alone or in combination, include: ethanedioic acid (oxalic acid), propanedioic acid (malonic acid), butanedioic acid (succinic acid), pentanedioic acid (glutaric acid), hexanedioic acid (adipic acid), heptanedioic acid (pimelic acid), octanedioic acid (suberic acid), nonanedioic acid (azelaic acid), decanedioic acid (sebacic acid), undecanedioic acid, dodecanedioic acid, tridecanedioic acid (brassylic acid), C₃₂-dimer fatty acid (commercial product from Cognis Corp., USA), benzene-1,2-dicarboxylic acid (phthalic acid), benzene-1,3-dicarboxylic acid (isophthalic acid) and, benzene-1,4-dicarboxylic acid (terephthalic acid).

The derivatives of the above mentioned carboxylic acids which may find use in the present invention are, in particular: i) C₁-C₁₂ alkyl mono- or diesters, of which C₄-C₁₂ alky mono or diesters may be specifically mentioned; ii) the corresponding dicarbonyl halides, in particular the dicarbonyl chlorides; and iii) the corresponding dicarboxylic anhydrides.

In accordance with a preferred embodiment of the present invention, at least one dicarboxylic acid compound (B) is selected from the group consisting of: hexanedioic acid; heptanedioic acid, octanedioic acid; nonanedioic acid; decanedioic acid; undecanedioic acid; dodecanedioc acid; and, the corresponding C₁ to C₁₂ alkyl diesters thereof. Good results have been attained with the aforementioned higher molecular weight diamines, where the dicarboxylic acid compound (B) meets the requirements of Formula (I):

(I) CₙH₂ₙ₊₁OC(CH₂)ₘCOOCₙH₂ₙ₊₁,

wherein: m is an integer from 6 to 10; and, n is an integer from 5 to 11.

### Enzymatic Catalyst (C)

Whilst any hydrolase enzyme [EC 3.x.x.x] which is capable of catalyzing a polycondensation reaction of diamine compound (A) and dicarboxylic acid compound (B) in aqueous medium may, in principle, be employed in the present invention, suitable enzymes (C) are generally esterases [EC 3.1.x.x], proteases [EC 3.4.x.x] and / or hydrolases which react with C-N bonds other than peptide bonds.

Of particular utility are: i) carboxylesterases [EC 3.1.1.1] derived from *Bacillus* sp., *Pseudomonas* sp., *Burkholderia* sp., *Mucor* sp., *Saccharomyces* sp., *Rhizopus* sp., *Thermoanaerobium* sp., porcine liver or equine liver; and; ii) lipases [EC 3.1.1.3] lipases derived from *Achromobacter* sp., *Aspergillus* sp., *Candida* sp., *Candida antarctica, Mucor* sp., *Penicilium* sp., *Geotricum* sp., *Rhizopus* sp, *Burkholderia* sp. *Pseudomonas* sp., *Pseudomonas* cepacia, *Thermomyces* sp., porcine pancreas or wheatgerms.

The use of more than one enzyme is not precluded by the present invention. Moreover, it is also possible to use these enzymes in free and/or immobilized forms.

In accordance with a preferred embodiment of the present invention, the enzymatic catalyst comprises a lipase derived from *Pseudomonas cepacia, rhizomucor miehei,* or *Candida antarctica* in free and/or immobilized form.

*Candida antarctica lipase* A is commercially-available-as a free enzyme and- as crosslinked enzyme aggregates (CLEA), immobilized on celite with sucrose, silicone particles, polypropylene beads and magnetic particles.

*Candida antarctica* lipase B is commercially available as the free enzyme or immobilized by absorption on macroporous beads known as Novozymes N435. The immobilization increases the thermostability of the lipase so that it can be used at temperatures up to 100°C. The N435 beads consist of a cross-linked resin of poly(methyl methacrylate) and have a diameter ranging from 0.3 to 0.9 mm. The catalyst has an average loading of 20% by weight with the lipase located in the outer 100 microns of the beads.

### Emulsifier (D)

The aqueous reaction medium comprises at least one emulsifier. Generally the reaction medium will contain from 0.005 to 20 parts by weight, preferably 0.1 to 10 parts by weight, based on 100 parts of the sum of the total amounts of diamine compounds (A) and dicarboxylic acid compound (B), of the emulsifier.

As used herein, the emulsifier(s) may be anionic, cationic or nonionic nature and will typically have relative molecular weights below 1000. Where mixtures of these emulsifiers are to be employed, the individual emulsifiers must be compatible with one another, which generally precludes combining anionic and cationic emulsifiers. An overview of suitable emulsifiers can be found in Houben-Weyl, Methoden der organischen Chemie, volume XIV/1, Makromolekulare Stoffe, Georg-Thieme-Verlag, Stuttgart, 1981, p 192 to 208.

Nonionic emulsifiers which can be used include but are not limited to: ethoxylated monoalkylphenols, dialkylphenols and trialkylphenols (EO units: 3 to 50, alkyl radical: C₄ to C₁₂); and, ethoxylated fatty alcohols (EO units: 3 to 80; alkyl radical: C₈ to C₃₆). Examples of such emulsifiers are the Lutensol® A, AO, AT, ON and TO brands available from BASF AG. Lutensol® AT50 may be specifically mentioned.

Suitable anionic emulsifiers include but are not limited to alkali metal and ammonium salts of: alkyl sulfates (alkyl radical: C₈ to C₁₂); sulfuric monoesters of ethoxylated alkanols (EO units: 4 to 30, alkyl radical: C₁₂ to C₁₈) and ethoxylated alkylphenols (EO units: 3 to 50, alkyl radical: C₄ to C₁₂); alkylsulfonic acids (alkyl radical: C₁₂ to C₁₈); and, alkylarylsulfonic acids (alkyl radical: C₉ to C₁₈). Further suitable anionic emulsifiers are disclosed in US Patent No. 4,269,749, the disclosure of which is herein incorporated by reference.

Suitable cationic emulsifiers are generally primary, secondary, tertiary or quaternary ammonium salts having a C₆-C₁₈ alkyl, C₆-C₁₈ alkylaryl or heterocyclic radical, alkanolammonium salts, pyridinium salts, imidazolinium salts, oxazolinium salts, morpholinium salts, thiazolinium salts and salts of amine oxides, quinolinium salts, isoquinolinium salts, tropylium salts, sulfonium salts and phosphonium salts. Specific examples of suitable, cationic surfactants may be found in McCutcheon's, Emulsifiers & Detergents, MC Publishing Company, Glen Rock, 1989.

### Optional Additional Components

The aqueous medium may comprise organic solvents in an amount up to 60 parts by weight, but preferably only in an amount up to 10 parts by weight, based on 100 parts by weight water. These solvents are often useful where the diamine compound (A) and the dicarboxylic acid compound (B) are highly soluble in water (e.g. ≥ 10g/l) under reactions conditions but the solvents used - and the amount thereof - should be selected carefully to ensure that the enzymes can retain their catalytic function. In general, these are organic solvents which enable the enzyme to retain its structural water and thus tend to be hydrophobic solvents as hydrophilic solvents mix easily with water and are therefore able to strip all the water from the catalyst. A measure for the hydrophobicity of the solvent is the logarithm of the partition coefficient (log P) of a solvent between octanol and water. Solvents with a log P value higher than 2 are preferred for use in the present invention.

Suitable solvents include but are not limited to: n-pentane, n-hexane, n-heptane, n-octane, n-nonane, n-decane, n-dodecane, n-tetradecane, n-hexadecane, n-octadecane, and the isomers thereof; cyclopentane; cyclohexane; toluene; ethyl benzene; cumene; o-, m- or p-xylene; mesitylene; fatty alcohols having from 10 to 28 carbon atoms such as n-dodecanol, n-tetradecanol, n-hexadecanol and isomers thereof, or cetyl alcohol; esters, for example fatty acid esters having from 10 to 28 carbon atoms in the acid moiety and from 1 to 10 carbon atoms in the alcohol moiety; or, esters of carboxylic acids and fatty alcohols having from 1 to 10 carbon atoms in the carboxylic acid moiety and from 10 to 28 carbon atoms in the alcohol moiety.

The aqueous medium may further be provided with one or more protective colloids, optimally in an amount of from 0.1 to 10 parts by weight based on the total weight of the diamine compound (A) ad the dicarboxylic acid compound (B). The colloids, acting as dispersants, should be compatible with the enzymatic catalyst and not deactivate that catalyst, which properties can be determined by simple experiments. A comprehensive description of suitable protective colloids can be found in Houben-Weyl, Methoden der organischen Chemie, Volume XIV/1, Makromolekulare Stoffe, Georg-Thieme-Verlag, Stuttgart, 1961, p. 411 to 420, the disclosure of which is herein incorporated by reference.

The aqueous medium should have a pH range in which the enzymatic catalyst operates optimally. Whilst this pH range may be determined by routine experimentation, the pH, as measured at 25°C, will generally be from 2 to 11, more usually from 3 to 9 and often from 6 to 8. As such, appropriate amounts of acids, bases and pH buffering substances may be added to the aqueous emulsion.

The reaction in the aqueous medium of the present invention may be effected or moderated by the addition thereto of further compounds having functional moieties: in that regard those compounds disclosed in paragraphs [0056] to [0068] of US Patent Application No. 2008/0275182 are incorporated herein by reference as optional components of the reaction medium.

In a preferred embodiment, at least one compound selected from the group consisting of valine, leucine isoleucine, threonine, methionine, phenylalanine, tryptophan, lysine, alanine, arginine, aspartic acid, cysteine, glutamic acid, glycine, histidine, proline, serine, tyrosine, asparagine and glutamine is added to the aqueous reaction medium. When added, the total amount of these amino acids in aqueous reaction medium may preferably be from 0.1 to 5 wt.%, based on the sum of the total amounts of the diamine compound (A) and the dicarboxylic acid compound (B). The advantage of this embodiment resides in the natural occurrence of these aminocarboxylic acids; when used in combination with the aforementioned fatty dimer diamines (A), a significant proportion of the raw materials for the polyamide synthesis can be derived from renewable sources.

### Methodology

Mini-emulsions are dispersions of critically stabilized oil droplets with a size between 40 and 500 nm prepared by shearing a system containing oil, water, a surfactant and a hydrophobe. Polymerizations in such miniemulsions can result in particles which have approximately the same size as the initial droplets (K. Landfester, Polyreactions in miniemulsions, Macromol. Rapid Comm. 2001, 896-936. K. Landfester, N. Bechthold, F. Tiarks, and M. Antonietti, Formulation and stability mechanisms of polymerizable miniemulsions. Macromolecules 1999, 32, 5222. K. Landfester, Recent Developments in Miniemulsions-Formation and Stability Mechanisms. Macromol. Symp. 2000,150,171).

In preparing the mini-emulsions of the present invention, at least a portion of the organic diamine compound (A), of the organic dicarboxylic acid compound (B) and of the emulsifier (D), as described above are first mixed using a homogenizer or the like to form an aqueous macroemulsion. The hydrolase catalyst (C) is not introduced at this point. A mini-emulsion is then formed by applying high shear forces to this mixture or macroemulsion, by sonication or by using high pressure homogenizers. The shear forces are generally applied for a period of from 1 to 10 to min, depending on the size of the reaction system and the precise homogenizer used. A time period of from 2 to 4 minutes has typically been found sufficient.

General methods for the preparation of aqueous miniemulsions from aqueous macroemulsions are of course known to those of ordinary skill in the art through, for example, the teaching of P.L. Tang et al. Journal of Applied Polymer Science, Vol. 43, pages 1059-1066 [1991].

The apparatus used to form the mini-emulsion is not intended to be limited: for example, it is expected that a Niro-Soavi High Pressure Homogenizer should provide repeatable results. However, good results have been consistently been obtained by sonication wherein an ultrasound homogenizer is operated at an amplitude of from 60-100% and preferably about 75-95%.

The disperse phase of the aqueous miniemulsions formed by sonication or otherwise should generally have an average droplet size of from 40 to 500nm. Preferably, that average droplet size (d_{z}) is from 50 to 400nm and more preferably from 100 to 300nm.

The enzymatic polymerization within the mini-emulsion is initiated by the addition of the enzymatic catalyst (C) thereto. The catalyst is added in an aqueous solution, preferably with one or more of the emulsifiers (D) present within the mini-emulsion and optionally with any fraction of the reacting compounds (A,B) not previously added. The total amount of water present in the mini-emulsion can obviously be moderated at this stage via the level of dilution of the catalyst (C).

After the addition of the enzyme in this way, the reaction mixture should typically be held, under stirring, at a temperature of from 20° to 80°C, e.g. from 45° to 60°C, and at an absolute pressure of from 0.8 to 5 bar, preferably from 0.9 to 1.5 bar e.g. 1 bar to allow the polymerziation to proceed. Whilst it is preferred that the process be carried out under an oxygen-free, inert gas atmosphere, it will be acknowledged that these are otherwise mild reaction conditions.

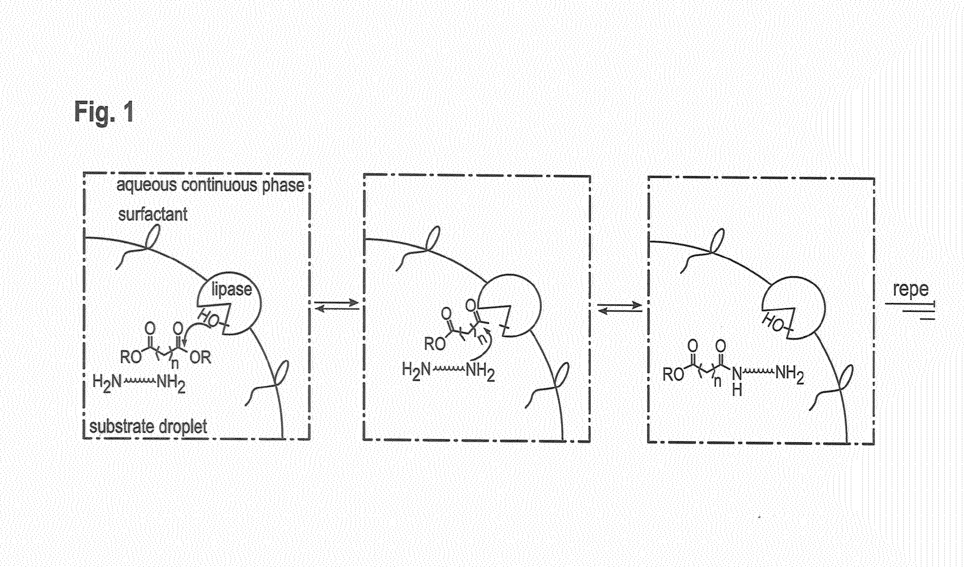

Without being bound by theory, it is postulated that the polycondensation reaction proceeds according to the scheme illustrated in Figure 1.

The diester, disposed within the stabilized substrate droplet, becomes associated with the active binding site of the lipase enzyme, wherein after the activation energy for the polycondensation reaction with that diamine, also disposed within the droplet, is lowered. The resultant amide is then released from the active binding site, allowing for the polycondensation reaction to be repeated and thus for oligomeric amides and polyamides to form.

After a period of from 0.5 to 10 days, the polymerization is preferably terminated by deactivating the enzyme (C) by either heating the obtained polyamide dispersion to temperatures above 90°C, changing the pH of that dispersion, or adding to that dispersion an effective amount of a deactivating compound which should at least inhibit the catalytic action of the enzyme (C). Where the polyamide dispersion is heated to temperatures above 100°C, boiling may have to be suppressed may increasing the ambient pressure.

Deactivating compounds may of course be enzyme specific but known deactivating compounds include nitrilotriacetic acid, ethylenediaminetetraacetic acid and alkali metal salts thereof, and anionic emulsifiers, such as sodium dodecylsulfate (SDS).

The dispersed polyamide particles obtainable by this process have average particles diameters (dz) in the range from 10 to 500nm, for example from 50 to 350nm. Concomitantly, the polyamides generally have a weight average molecular weight (Mw) of from 5000 to 1,000,000 g/mol as determined by Gel Permeation Chromatography in accordance with DIN 55672-1.

Where required, the aqueous dispersions of polyamide particle may be dried - by freeze or spray drying for instance - to form a polyamide powder.

The following examples are illustrative of the present invention and are not intended to limit the scope of the invention in any way.

### Examples

### i) Materials

The following materials were employed in the Examples.

| | |
|---|---|
| Lutensol AT50 | Non-ionic surfactant based on alcohol alkoxylates available from BASF. |
| Priamine 1074 | Dimer diamine available from Croda Europe Ltd. |
| Jeffamine D-230 | Polyetheramine (difunctional, primary amine) of approximate average molecular weight 230 available from Huntsman Corporation. |
| Jeffamine D-400 | Polyetheramine (difunctional, primary amine) of approximate average molecular weight 430 available from Huntsman Corporation |
| Jeffamine ED-600 | Aliphatic polyether diamine derived from a propylene oxide-capped polyethylene glycol of approximate average molecular weight 600 available from Huntsman Corporation |
| Jeffamine ED-900 | Aliphatic polyether diamine derived from a propylene oxide-capped polyethylene glycol of approximate average molecular weight 900 available from Huntsman Corporation. |
| PSL | Lipase *pseudomonas cepacia* available from Amano. |
| Novozyme 435 | Immobilized lipase B from *candida antartica,* available from Novozymes A/S. |
| Novozyme 735 | Immobilized lipase A from *candida antartica,* available from Novozymes A/S. |
| Lipase L51 | Novozyme 51032, available from Novozymes A/S. |
| Lypozyme CALB L | Lipase from *rhizomucor miehei,* available from Novozymes A/S. |
| CALA | Lipase A from *candida antartica,* available from Novozymes A/S. |
| CALB | Lipase A from *candida antartica,* available from c-Lecta GmbH. |

### ii) Analysis

The following analytical techniques were used in the Example.

Molecular weights of obtained polymers were determined by end group analysis by means of ¹H NMR or GPC analysis.

¹H nuclear magnetic resonance (NMR) spectroscopy was recorded on a Bruker Avance400 spectrometer using deuterated chloroform or deuterated trifluroacetic acid as a solvent.

Gel Permeation Chromatography (GPC) was performed on an Alliance HPLC system equipped with refractive index detector Waters 2414 and Styragel columns HR5, HR3 and HR1. The products were dissolved in tetrahydrofuran (THF) to form a 1.0 wt.% solution. The solution was heated at 50°C where a part of the product was insoluble at room temperature. After filtration through a syringe filter, the filtrate was injected into the GPC equipment using THF-diethylamine as an eluent at 50°C. Molecular weights were evaluated by extrapolation calibrated by polystyrene.

IR Spectroscopy was performed on a Bruker IFS66V/S spectrometer.

Particle sizes were measured before and after the enzymatic reaction by means of dynamic light scattering (DLS) at low concentration on a Submicron Particle Sizer NICOMP™ 380 available from PSS NICOMP Particle Sizing Systems.

### iii) Experimental

Sebatic diester substrates (CₙH₂ₙ₊₁OOC(CH₂)₈COOCₙH₂ₙ₊₁, where n is from 5 to 11) were individually synthesized from dichloro sebacate with alcohols in diethyl ether in the presence of pyridine as follows:
Aliphatic alcohol (C₅-C₁₁), 210 mmol) and pyridine (210 mmol) were dissolved in diethyl ether (200 ml). An ether solution of dichloro sebacate (105 mmol in 20ml) was added dropwise over 20 minutes at 0°C and stirred for 30 minutes at the same temperature.

After further stirring for 4 hours at room temperature, the reaction mixture was poured into ice water and then extracted with diethyl ether.

An organic layer was dried over MgSO₄ and evaporated to give colorless oil or white solid depending on the chain length of the alcohol. The residual oil was purified by silica column chromatography (at a ratio of hexane to ethyl acetate of 9:1) and white solid was washed with water and ethanol.

The thus obtained sebacic diesters were identified by ¹H nuclear magnetic resonance (NMR).

The enzymatic polycondensation of diester with diamine to synthesize polyamide nanoparticles in aqueous mini-emulsion was carried out using the following procedure.

To an aqueous solution of Lutensol AT50 (1.0wt.%, 19g) were added diester (5.0 mmol), diamine (5.0 mmol) and hexadecane (0.16g). A pre-emulsion was prepared by vigorous stirring at 16000 rpm using Ultra Turrax T25 high-shear mixer. Whilst cooling the vessel on an ice-water bath, the pre-emulsion was then successfully ultrasonicated using a Branson Digital Sonifier Model 450-D at 90% amplitude for 9 periods of 20 seconds, each period being separated by a 5 second interval, to form an aqueous mini-emulsion having an average particles diameters (dz) in the range from 50 to 500nm.

The enzymatic polymerization was initiated by addition of lipase (200mg) in a 1.0 wt.% Lutensol AT50 solution (1.0g) and the reaction mixture was stirred at 55°C for 24 hours or 1 week. The enzyamatic reaction was then terminated by the addition of SDS (50mg) in water (0.50 ml) and then immediately lyophilized or separated after addition of CHCl₃.

The residues were characterized by means of ¹H nuclear magnetic resonance (NMR) and Gel Permeation Chromatography.

### iv) Results

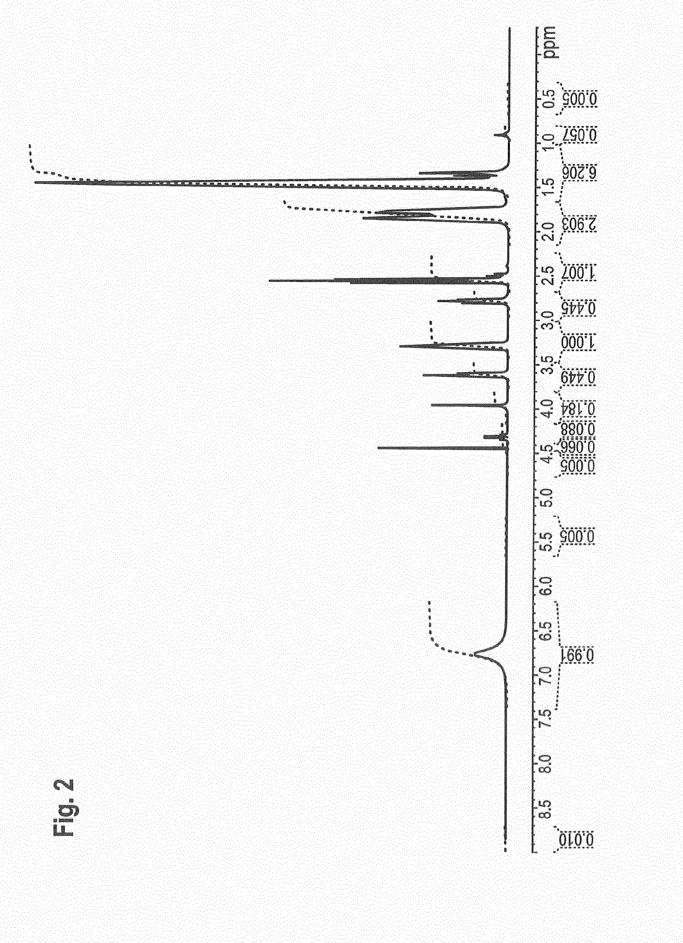

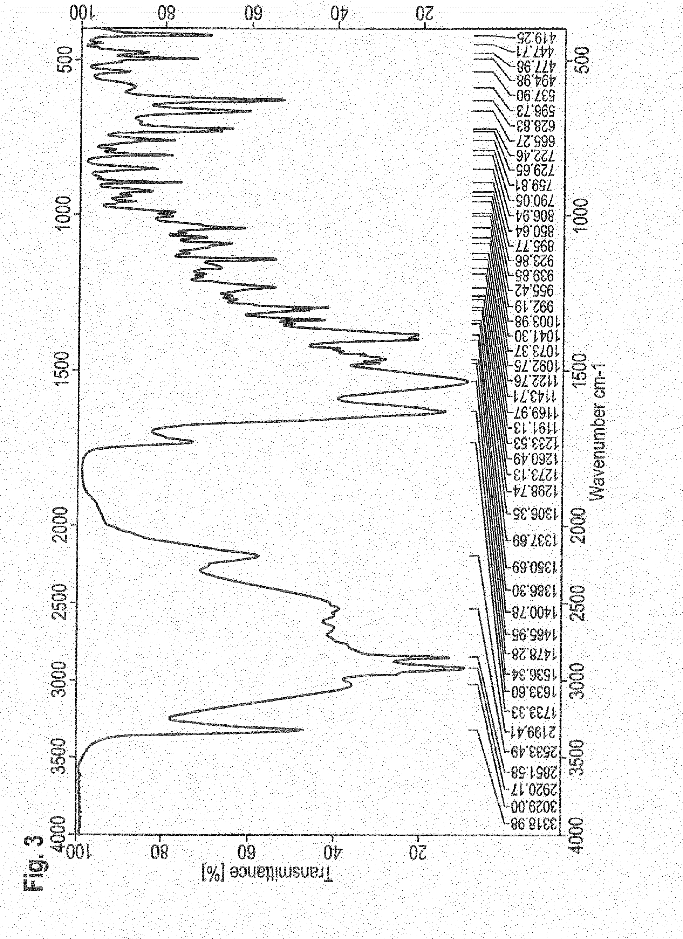

As shown in the drawings appended hereto, Fig. 2 is a ¹H Nuclear Magnetic Resonance (NMR) spectrum of the crystalline product of a Lipase PS (as described in Structure 1997, Vol. 5 No. 2, 187 - 202) catalyzed polycondensation of diethyl sebacate with 1,8-diaminooctane as obtained by the above experimental method. This crystalline product was also characterized by its IR spectrum, as illustrated in Fig. 3 appended hereto.

Table 1 below details the effect of changing the alkyl group of di(alkyl) sebacate upon the yield and molecular weight of the polyamides obtained in the lipase-catalyzed Lipase PS (as described in Structure 1997, Vol. 5 No. 2, 187 - 202) catalyzed p polycondensation reaction with 1,8-diaminooctane in aqueous mini-emulsion.

**Table 1**

| n | Partial Solubility of CₙH₂ₙ₊₁OH in water (g/l)^{a} | Salt Yield (%) | Polyamide Yield (%) |
|---|---|---|---|
| 2 | ∞ | 45 | 11 |
| 4 | 115 | 31 | 3.4 |
| 5 | 32 | 25 | 2.5 |
| 6 | 7.9 | 20 | 2.9 |
| 7 | 2.5 | 14 | 1.8 |
| 8 | 0.3 | 11 | 0.37 |

| | | | |
|---|---|---|---|
| ^{a}Data obtained from Handbook of Chemistry and Physics 86^{th} Edition, CVC Press. | | | |

Table 2 below details the effect of changing the reaction temperature, diar reaction conditions upon the yield and molecular weight of the polyamide Lipase-catalyzed polycondensation reaction with diethyl sebacate (n =2) emulsion. The enzymatic reactions recorded in Table 2 were carried out at E except where otherwise stated in the "Catalyst (conditions)" column.

**Table 2**

| **No.** | **Diamine** | **Catalysts (Conditions)** | **Average Particle Size (dz) Before / After reaction** | | **Polyamid Yield (%)** |
|---|---|---|---|---|---|
| 1 | Priamine 1074 | PSL | 194/234 | | 3.9 |
| 2 | Priamine 1074 | Novozyme 435 | | | 12 |
| 3 | Priamine 1074 | Novozyme 435 (1 week) | | | 12 |
| 4 | Priamine 1074 | Novozyme 435 (35°C) | | | 12 |
| 5 | Priamine 1074 | Novozyme 435 (45°C) | | | 10 |
| 6 | Priamine 1074 | Novozyme 435 (65°C) | | | 10 |
| 7 | Priamine 1074 | Novozyme 435 (75°C) | | 11 | 3900 |
| 8 | Priamine 1074 | Novozyme 435 (85°C) | | 8 | 4300 |
| 9 | Priamine 1074 | CALB | | 19 | 4100 |
| 10 | Priamine 1074 | CALA | | 6.8 | 4700 |
| 11 | Priamine 1074 | CRL1 | | 7.2 | 4600 |
| 12 | Priamine 1074 | Lypozyme CALB L | | 22 | 3900 |
| 13 | Priamine 1074 | Novozyme 735 | | 7.1 | 4600 |
| 14 | Priamine 1074 | Novozyme 51032 | | 21 | 4200 |
| 15 | Jeffamine D400 | CALB | 265 / 323 | 48 | 3300 |
| 16 | Jeffamine ED600 | CALB | 210 / 267 | 24 | 3600 |
| 17 | Jeffamine ED900 | CALB | 135 / 313 | 11 | 4200 |

## Claims

1. A process for preparing an aqueous polyamide dispersion, said process comprising reacting in an aqueous medium:
a) an organic diamine compound (A) and
b) an organic dicarboxylic acid compound (B),
in the presence of
c) at least one hydrolase enzyme (C) which catalyzes a polycondensation reaction of the diamine compound (A) and dicarboxylic acid compound (B) and
d) an emulsifier (D)
said process being **characterized in that** at least a fraction of the diamine compound (A) and the dicarboxylic acid compound (B) are provided in the aqueous medium as a disperse phase having an average droplet diameter of from 40 to 500nm.

2. The process according to claim 1, wherein the organic diamine compound (A) is selected from the group consisting of fatty dimer amines and poly(alkylene oxide) diamines having terminal, primary amine groups.

3. The process according to claim 1 or claim 2, wherein the dicarboxylic acid compound (B) is selected from the group consisting of: hexanedioic acid; heptanedioic acid, octanedioic acid; nonanedioic acid; decanedioic acid; undecanedioic acid; dodecanedioc acid; and, the corresponding C₄ to C₁₂ alkyl diesters thereof.

4. The process according to claim 3, wherein the dicarboxylic acid compound (B) has the general Formula:
(I) CₙH₂ₙ₊₁OOC(CH₂)ₘCOOCₙH₂ₙ₊₁,
wherein: m is an integer from 6 to 10; and, n is an integer from 5 to 11.

5. The process according to any one of claims 1 to 4, wherein the molar ratio of the dicarboxylic acid compound (B) to the organic diamine compound (A) is from (0.5-1.5): 1, preferably from (0.8-1.2): 1, and more preferably from (0.95-1.05):1, for example 1:1.

6. The process according to any one of claims 1 to 5, wherein at least one hydrolase enzyme (C) is an esterase [EC 3.1.x.x].

7. The process according to claim 6, wherein said esterase is selected from carboxylesterases [EC 3.1.1.x] and lipases [EC 3.1.1.3].

8. The process according to any one of claims 1 to 7, wherein the total amount of hydrolase enzyme (C) in the aqueous medium is from 0.001 to 40% by weight, preferably from 0.1 to 15% by weight and more preferably from 0.5 to 8% by weight, based on the total amounts of diamine compound (A) and dicarboxylic acid compound (B) in said medium.

9. The process according to any one of claims 1 to 8, wherein the aqueous medium further comprises up to 10 parts by weight, based on 100 parts by weight of water, of an organic solvent having a logarithm of the octanol-water partition coefficient (log P) greater than 2.

10. An aqueous polyamide dispersion obtainable by the process according to any one of claims 1 to 9.

11. A mini-emulsion comprising:
an emulsifier (D);
a disperse phase comprising an organic diamine compound (A) and a carboxylic acid compound (B); and
a continuous phase comprising water
wherein said disperse phase has an average droplet diameter of from 40 to 500nm.

12. A mini-emulsion according to claim 11, wherein the disperse phase has an average diameter of from 100 to 300nm.

13. A mini-emulsion according to claim 11 or claim 12, wherein
said organic diamine compound (A) is selected from the group of aliphatic diamines consisting of 1,6-diaminohexane, 1,7-diaminoheptane, 1,8-diaminooctane, 1,9-diaminononane, 1,10-diaminodecane, 1,11-diaminoundecane, 1,12-diaminododecane and, mixtures thereof; and
the dicarboxylic acid compound (B) has the general Formula (I) CₙH₂ₙ₊₁OOC(CH₂)ₘCOOCₙH₂ₙ₊₁, in which m is an integer from 6 to 10 and n is an integer from 5 to 11.

14. A mini-emulsion according to claim 11 or claim 12, wherein:
said organic diamine compound (A) is selected from the group consisting of fatty dimer amines and poly(alkylene oxide) diamines having terminal, primary amine groups; and
the dicarboxylic acid compound (B) has the general Formula (I) CₙH₂ₙ₊₁OOC(CH₂)ₘCOOCₙH₂ₙ₊₁, in which m is an integer from 6 to 10 and n is an integer from 5 to 11.

15. A mini-emulsion according to any one of claims 11 to 14 further comprising a hydrolase enzyme (C).
